(19) Europäisches Patentamt
European Patent Office
Office européen des brevets

(11) **EP 4 779 010 A1**

(12) **EUROPEAN PATENT APPLICATION**
published in accordance with Art. 153(4) EPC

(43) Date of publication:
**22.07.2026 Bulletin 2026/30**

(21) Application number: **24865056.6**

(22) Date of filing: **10.07.2024**

(51) International Patent Classification (IPC):
*C12M 1/00* (2006.01)      *C12M 3/00* (2006.01)
*C12N 1/00* (2006.01)      *C12N 5/07* (2010.01)

(52) Cooperative Patent Classification (CPC):
**C12M 1/00; C12M 3/00; C12N 1/00; C12N 5/06**

(86) International application number:
**PCT/JP2024/024976**

(87) International publication number:
**WO 2025/057549 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.09.2023 JP 2023149200**

(71) Applicant: **HITACHI HIGH-TECH CORPORATION
Tokyo 105-6409 (JP)**

(72) Inventors:
• **KOSAKA, Junya
Tokyo 105-6409 (JP)**
• **ISHIGAMI, Yukihiko
Tokyo 105-6409 (JP)**

(74) Representative: **MERH-IP Matias Erny Reichl
Hoffmann
Patentanwälte PartG mbB
Paul-Heyse-Straße 29
80336 München (DE)**

(54) **CELL CULTURE EXPERIMENT SYSTEM, CELL CULTURE EXPERIMENT METHOD, AND PROGRAM**

(57)    A period until an appropriate environmental parameter is obtained is shortened in a cell culture experiment. The control device sets a recipe for each sample of a cell and controls a robot device such that culture experiments are performed according to the recipes. A database device sequentially stores intermediate progress data for each sample in a progress of one culture experiment in which a culture result is determined. The recipe determination device analyzes a tendency of culture for each sample based on the intermediate progress data, and calculates a posterior probability representing a degree of possibility that a value of each of a plurality of environmental parameters that constitute the set recipe causes future successful culture, based on an analysis result of the tendency and a difference in the time-series intermediate progress data of the sample. The recipe determination device uses one or more of the environmental parameters having a relatively high posterior probability as a verification parameter, and corrects the recipe by changing a value of the verification parameter for each sample. The control device controls the robot device such that the experiments are continued according to the corrected recipes.

[FIG. 1]

**Description**

Technical Field

The present invention relates to a cell culture experiment system, a cell culture experiment method, and a program.

Background Art

In a life science industry, modalities for regenerative medicine and cell production are still in early days. There are various disturbance factors before a researcher obtains a desired normal cell, and it is necessary to repeat an enormous number of experiments at once to find appropriate intermediate environmental parameters. A set of environmental parameters for producing or culturing cells is also called a recipe.

As a general method used in an experiment for producing or culturing such cells, a Bayesian experimental design is known. For example, PTL 1 describes that, in a cell culture experiment, one or more types of execution parameter items that may affect an evaluation performance result and a search range thereof are estimated based on a given characteristic extraction rule from one or more types of execution performance results and evaluation performance results in the past, and that, based on the one or more types of execution performance results and evaluation performance results in the past, a plurality of variable parameter values in the search range are generated in accordance with Bayesian optimization or the like.

Citation List

Patent Literature

PTL 1: JP2023-035238A

Summary of Invention

Technical Problem

In the Bayesian experimental design, it is possible to reduce the number of experiments to be performed, that is, an amount of data necessary for the experiment, and to improve efficiency of the experiment by utilizing an experiment result for a next experimental design. However, in the Bayesian experimental design, the next experimental design cannot be made until the experiment result is obtained, and a time required for the experiment itself cannot be shortened. For example, it takes a long time, such as about four months, from the start of cell culture until a result is determined as a normal cell, and this period cannot be shortened.

For this reason, an accurate result is obtained and the appropriate intermediate environmental parameters are learned after four months, and it generally takes another four months before a next result is obtained using the learned intermediate environmental parameters, which significantly reduces research efficiency. Even if a search period is substantially shortened by conducting an enormous number of experiments at once, each experiment is required to be started according to priorities of the experiments.

An object of the invention is to shorten a period until an appropriate environmental parameter is obtained in a cell culture experiment.

Solution to Problem

One of representative embodiments of the invention is a cell culture experiment system including:

a recipe determination device configured to set a recipe to be applied to each of a plurality of samples of a cell to be cultured;
a robot device;
a control device configured to control an operation of the robot device such that the robot device performs a culture experiment on each of the plurality of samples according to the set recipe; and
a database device configured to store intermediate progress data for each of the samples in a time series manner in a progress of one culture experiment in which a culture result of each of the plurality of samples is determined, in which the recipe determination device

analyzes a tendency in culture for each sample based on the intermediate progress data for each sample stored in the database device,

calculates, for each sample, a posterior probability representing a degree of possibility that a value of each of a plurality of environmental parameters that constitute the set recipe causes future successful culture of the sample, based on an analysis result of the tendency in the culture of the sample and a difference in the time-series intermediate progress data of the sample,

determines one or more of the environmental parameters having a relatively high calculated posterior probability as a verification parameter, and

corrects the recipe for each sample by changing a parameter value of the determined verification parameter, and

the control device controls the operation of the robot device such that the robot device continues to perform the plurality of culture experiments according to the corrected recipes.

One of representative embodiments of the invention is a cell culture experiment method including:

setting, by a recipe determination device, a recipe to be applied to each of a plurality of samples of a cell to be cultured;

controlling, by a control device, an operation of a robot device such that the robot device performs a culture experiment on each of the plurality of samples according to the set recipes; and

storing, by a database device, intermediate progress data for each of the samples in a time series manner in a progress of one culture experiment in which a culture result of each of the plurality of samples is determined, in which the recipe determination device

analyzes a tendency in culture for each sample based on the intermediate progress data for each sample stored in the database device,

calculates, for each sample, a posterior probability representing a degree of possibility that a value of each of a plurality of environmental parameters that constitute the set recipe causes future successful culture of the sample, based on an analysis result of the tendency in the culture of the sample and a difference in the time-series intermediate progress data of the sample,

determines one or more of the environmental parameters having a relatively high calculated posterior probability as a verification parameter, and

corrects the recipe for each sample by changing a parameter value of the determined verification parameter, and

the control device controls the operation of the robot device such that the robot device continues to perform the plurality of culture experiments according to the corrected recipes.

One of representative embodiments of the invention is a program causing one or more computers or processors to function as:

a recipe determination device configured to set a recipe to be applied to each of a plurality of samples of a cell to be cultured;

a control device configured to control an operation of a robot device such that the robot device performs a culture experiment on each of the plurality of samples according to the set recipe; and

a database device configured to store intermediate progress data for each of the samples in a time series manner in a progress of one culture experiment in which a culture result of each of the plurality of samples is determined, in which the recipe determination device

analyzes a tendency in culture for each sample based on the intermediate progress data for each sample stored in the database device,

calculates, for each sample, a posterior probability representing a degree of possibility that a value of each of a plurality of environmental parameters that constitute the set recipe causes future successful culture of the sample, based on an analysis result of the tendency in the culture of the sample and a difference in the time-series intermediate progress data of the sample,

determines one or more of the environmental parameters having a relatively high calculated posterior probability as a verification parameter, and

corrects the recipe for each sample by changing a parameter value of the determined verification parameter, and

the control device controls the operation of the robot device such that the robot device continues to perform the plurality of culture experiments according to the corrected recipes.

Advantageous Effects of Invention

According to the representative embodiments of the invention, there is provided a technique capable of shortening a period until an appropriate environmental parameter is obtained in a cell culture experiment.

Brief Description of Drawings

[FIG. 1] FIG. 1 is a diagram schematically showing an example of a configuration of a cell culture experiment system according to Embodiment 1.
[FIG. 2] FIG. 2 is a diagram showing an appearance example of a main part of the cell culture experiment system according to Embodiment 1.
[FIG. 3] FIG. 3 is a diagram showing a configuration example of a computer.
[FIG. 4] FIG. 4 is a diagram showing a configuration example of data stored in a database device.
[FIG. 5] FIG. 5 is a diagram (part 1) showing an example of a flow of processing in the cell culture experiment system according to Embodiment 1.
[FIG. 6] FIG. 6 is a diagram (part 2) showing an example of the flow of processing in the cell culture experiment system according to Embodiment 1.
[FIG. 7] FIG. 7 is a diagram (part 3) showing an example of the flow of processing in the cell culture experiment system according to Embodiment 1.
[FIG. 8] FIG. 8 is a diagram showing an example of an operation of a robot device according to operation data.
[FIG. 9] FIG. 9 is a diagram showing a process of peeling adherent cells in a cell culture process.
[FIG. 10] FIG. 10 is a diagram showing a state in which a plurality of devices are operated by a common clock signal.
[FIG. 11] FIG. 11 is a diagram showing an example of a plurality of pieces of video data that are a part of intermediate progress data.
[FIG. 12] FIG. 12 is a diagram showing a force sensor as an example of a sensor unit of a detection device.
[FIG. 13] FIG. 13 is a diagram showing a specific case related to contact of an object.
[FIG. 14] FIG. 14 is a diagram showing an example of data of pressure applied to the object in processes, each of which is a part of the intermediate progress data.
[FIG. 15] FIG. 15 is a diagram showing an example of a part of the intermediate progress data.
[FIG. 16] FIG. 16 is a graph showing an example of a bar graph representing a distribution of a likelihood function.
[FIG. 17] FIG. 17 is a diagram showing an example of a table showing a likelihood function that represents a relationship between a prior probability distribution and likelihood.
[FIG. 18] FIG. 18 is a graph showing an example of a bar graph of a posterior probability.

Description of Embodiments

<History of Study Performed by Inventors>

Before the embodiments of the invention are described, problems in the case of applying a Bayesian experimental design to cell culture will be described using specific examples.
In a general Bayesian experimental design, an experiment is performed by using a posterior result. For example, it is assumed that there are newly developed cultured cells X. In order to verify an effect of X, it is assumed that results are verified for 20 randomly selected specimens out of a total of 40 specimens assumed in advance. The 20 specimens are referred to as a verification group. Further, false cells are introduced into the remaining 20 specimens. The false cells are cells that are not expected to have any effect. These 20 specimens are referred to as an efficiency improvement group. As a result, 4 specimens in the verification group develop into cancer (16 specimens are successful), and 16 specimens in the efficiency improvement group develop into cancer (4 specimens are successful).
If the cultured cells X can be determined to be effective from the result, a rational determination can be made with 50% efficiency in man-hours, without having to verify all the 40 specimens. First, a posterior probability distribution is obtained by the following Bayes theorem.

$$f(\pi|y) \text{ (POSTERIOR PROBABILITY DISTRIBUTION)} = \frac{f(\pi) \text{ (PRIOR PROBABILITY DISTRIBUTION)} f(y|\pi) \text{ (LIKELIHOOD FUNCTION)}}{f(y) \text{ (GENERALIZED CONSTANT)}} \quad \cdots \ (1)$$

The probability is essentially a continuous value, but for simplicity, it is assumed that a value of p (probability that the specimens belong to the verification group when one of the specimens develops into cancer) takes discrete values in increments of 0.1, such as 0.1, 0.2, 0.3, ..., 0.9 (that is, a discrete probability distribution is used).

In a Bayesian estimation, a subjective probability (uniform distribution) is used as a prior probability distribution. Generally used Bayesian statistics is, in brief, to obtain a posterior probability distribution (Posterior) from a prior probability distribution (Prior) and likelihood.

. As data increases, the posterior probability distribution becomes more accurate.

. Once the data becomes large enough, the prior probability distribution becomes almost completely fine.

In the Bayesian statistics, it is known that if data is sufficiently collected, a proportion of the prior probability distribution in the calculation is small enough to be ignored.

In a first stage, it is assumed that it is not known at all how effective the cultured cell X is. Therefore, nine models with p = 0.1 to p = 0.9 are considered equally likely, and the prior probabilities are uniformly set to 0.11 (0.12 is set only when p = 0.5 in order to make a sum 1).

f(0.1) = 0.11 (* it means that the probability when p = 0.1 is 0.11. The same applies hereinafter).

f(0.2) = 0.11
f(0.3) = 0.11
f(0.4) = 0.11
f(0.5) = 0.12
f(0.6) = 0.11
f(0.7) = 0.11
f(0.8) = 0.11
f(0.9) = 0.11

This probability distribution indicates a uniform distribution meaning that all the prior probabilities are the same, that is, in the cases of p = 0.1 to p = 0.9, the prior probabilities are all equally likely. (Strictly speaking, in order to make the sum 1, only when p = 0.5, 0.12 is set).

Next, a likelihood function is obtained. For example, the likelihood when a success rate is 0.5 is the same as a conditional probability that the number of successes is 4 when a trial is performed 20 times, and is obtained by the following calculation.

$$ P(k = 4|n = 20, p = 0.5) = \binom{20}{4}(0.5)^4(1 - 0.5)^{16} \approx 0.0046 \qquad \cdots \ (2) $$

When the same calculation is performed for p = 0.1 to 0.9, respective likelihoods are obtained as follows.

p = 0.1: 0.08977882814987155
p = 0.2: 0.21819940194609996
p = 0.3: 0.13042097437387037
p = 0.4: 0.03499079040415815
p = 0.5: 0.004620552062988271
p = 0.6: 0.0002696861504765946
p = 0.7: 5.007558331512445e-06
p = 0.8: 1.3005697843199945e-08
p = 0.9: 3.1788044999999754e-13

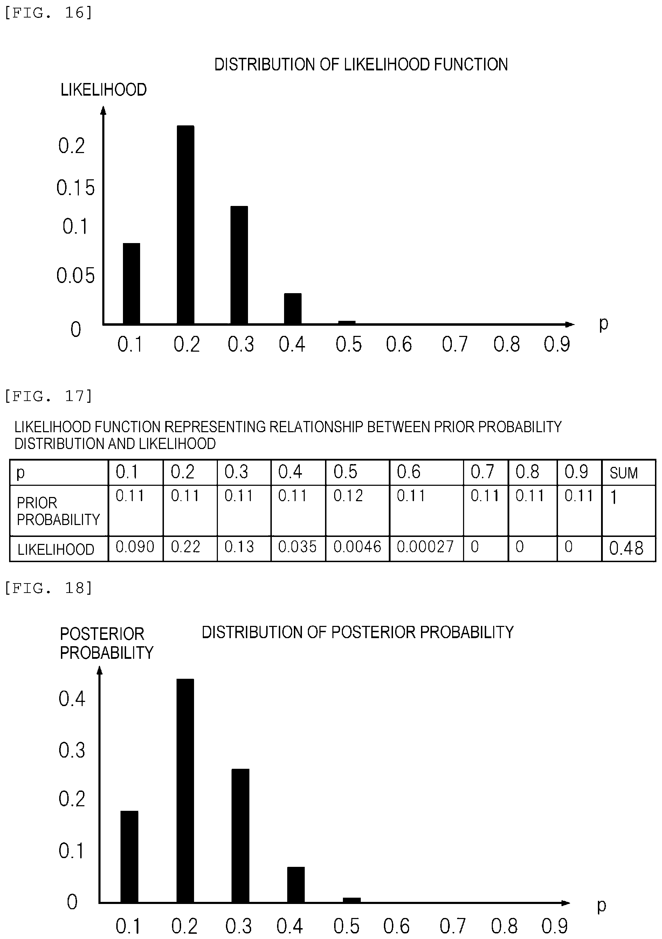

FIG. 16 is a graph showing an example of a bar graph representing a distribution of the likelihood function. When these described above are put together into a graph, the distribution of the likelihood function as shown in FIG. 16 is obtained.

A normalization constant is a constant for adjusting, to 1, the sum of probabilities of the posterior probability distribution to be obtained from this. This is a sum of products of the prior probability distribution and a corresponding likelihood function that have been calculated so far.

FIG. 17 is a diagram showing an example of a table showing the likelihood function that represents a relationship between the prior probability distribution and the likelihood. First, when the likelihood function representing the relationship between the prior probability distribution and the likelihood are summarized again in a table, the table as shown in FIG. 17 is obtained.

Since the normalization constant is obtained by multiplying these values one by one for each column and adding the products, $0.11 \times 0.0898 + 0.11 \times 0.2182 + 0.11 \times 0.1304 + 0.11 \times 0.035 + 0.12 \times 0.0046 + 0.11 \times 0.0003 + 0.11 \times 0.00 + 0.11 \times 0.00 \approx 0.053$ is obtained.

As described above, the posterior probability distribution can be obtained using the Bayes theorem. For example, the posterior probability when p = 0.5 is obtained as follows.

$$P(n = 20, p = 0.5 | k = 4) = \frac{P(p = 0.5)P(k = 4 | n = 20, p = 5)}{P(k = 4)}$$

$$= \frac{0.12 \cdot 0.0046}{0.053} \approx 0.0105 \qquad \cdots \ (3)$$

This is the posterior probability of the model of p = 0.5. This indicates that "the probability that a cancer incidence is 0.5 when there are four cancer cases in 20 trials" is only 1%.

Similarly, the remaining eight models of p = 0.1 to p = 0.9 are calculated as follows.

P(k = 4|n = 20, p = 0.1): 0.1875450876140688
P(k = 4|n = 20, p = 0.2): 0.45581154041134897
P(k = 4|n = 20, p = 0.3): 0.27244522533562093
P(k = 4|n = 20, p = 0.4): 0.07309463697920575
P(k = 4|n = 20, p = 0.5): 0.010529656158194935
P(k = 4|n = 20, p = 0.6): 0.0005633657039385877
P(k = 4|n = 20, p = 0.7): 1.0460628472988208e-05
P(k = 4|n = 20, p = 0.8): 2.716848495074202e-08
P(k = 4|n = 20, p = 0.9): 6.640420472689636e-13

FIG. 18 is a graph showing an example of a bar graph of the posterior probability. When these posterior probabilities described above are drawn in a bar graph, the graph as shown in FIG. 18 is obtained.

According to the graph of the posterior probability distribution, there is an almost 100% probability that p < 0.5 (the probability of canceration is 50% or less) (in practice, from the graph in FIG. 18, it is found that 0.188 + 0.456 + 0.272 + 0.0731 = 0.989). In short, the possibility that the cultured cells X are effective is 98.9%.

Among these probabilities, the probability of p = 0.2 stands out at 0.456. From this, it can be seen that the probability (0.2) that a cancerous cell is a specimen from the verification group is most likely to be 1/4 of the probability (0.8) that the cancerous cell is a specimen from the efficiency improvement group (that is, when the cultured cells X are applied, the cancer incidence is most likely to be 1/4).

The above is a case of the generally used Bayesian experimental design, and in this case, it can be said that a reasonable result can be derived even if the number of specimens is 50% (that is, half the man-hours).

However, a problem in a culture environment to which the invention is applied is that it takes a long time (for example, four months) from the start of the culture until the result is determined as normal cells (or cancerous cells). Therefore, an accurate result is obtained and an appropriate recipe, that is, an intermediate environmental parameter set, is derived through learning after four months, and it generally takes another four months before a next result is obtained using the intermediate environmental parameter set derived through the learning. This is a factor that significantly reduces research efficiency.

In the Bayesian experimental design, since it is necessary to set all the probability distributions at once, it is necessary to perform an enormous number of experiments at once and obtain enormous results. However, in practice, even if the search period is substantially shortened by conducting the enormous number of experiments at once, the experiments are required to be started sequentially according to priorities of the experiments, that is, the results are required to be compiled in order, which is a problem.

As a result of intensive studies in view of the above circumstances, the inventors have created the invention. Hereinafter, embodiments of the invention will be described in detail with reference to the drawings.

In the drawings, the same elements are denoted by the same reference signs in principle, and a repeated description thereof is omitted. When there are a plurality of identical or similar components, the same reference numerals may be assigned with different subscripts. In the drawings, expressions of each component may not represent an actual position, size, shape, range, and the like in order to facilitate understanding of the invention, and the invention is not necessarily limited to the position, size, shape, range, and the like disclosed in the drawings. Expressions such as identification information, an identifier, an ID, a name, and a number of various kinds of data and information can be mutually replaced.

Functional blocks, circuits, computers, processing units, and the like which constitute a device or a system may be implemented by an electronic circuit or the like in which electronic components are combined, or may be implemented by an information processing device such as a processor or a computer including a processor executing a predetermined program.

The processor is implemented by semiconductor devices such as a central processing unit (CPU), a graphics processing unit (GPU), or a micro controller unit (MCU). The processor is implemented by a device or a circuit capable of performing a predetermined calculation. The processing is not limited to software program processing, and can be executed by a dedicated circuit. The dedicated circuit may be a field programmable gate array (FPGA) or an application specific integrated circuit (ASIC), or the like.

The program may be installed as data in a target computer in advance, or may be distributed as data from a program source to a target computer and installed. The program source may be a program distribution server on a communication network or a non-transitory computer-readable storage medium. The program may include a plurality of program modules. A computer system may include a plurality of devices.

(Embodiment 1)

<Configuration of Cell Culture Experiment System>

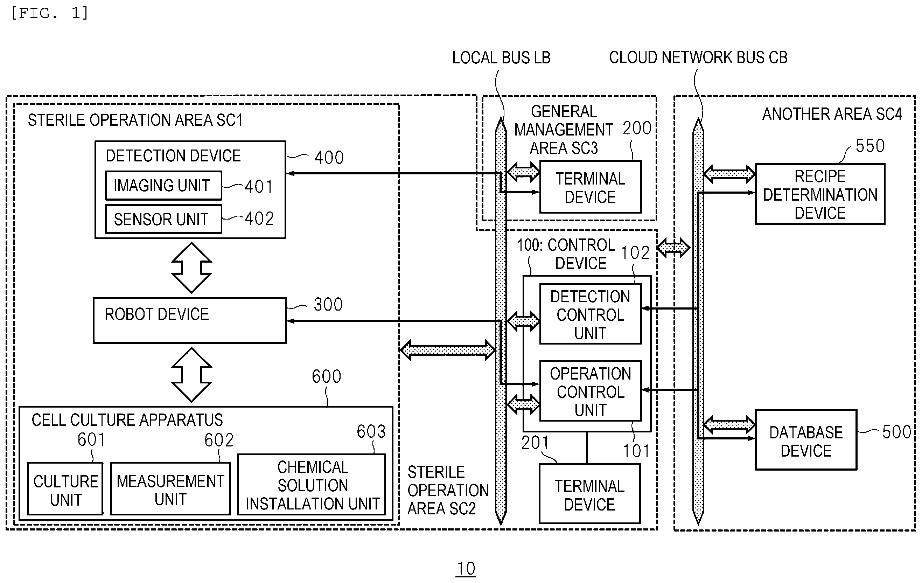

FIG. 1 is a diagram schematically showing an example of a configuration of a cell culture experiment system according to Embodiment 1. As shown in FIG. 1, a cell culture experiment system 10 according to Embodiment 1 includes devices placed in an sterile operation area SC1 where a grade A is assumed, devices placed in an sterile operation area SC2 where a grade B/C is assumed, a device placed in a general management area SC3, and devices placed in another area SC4.

A robot device 300, a detection device 400, and a cell culture apparatus 600 are provided in the sterile operation area SC1. A control device 100 and a terminal device 201 are provided in the sterile operation area SC2. A terminal device 200 is provided in the general management area SC3. A recipe determination device 550 and a database device 500 are provided in the other area SC4.

The devices provided in the sterile operation area SC1 and the devices provided in the sterile operation area SC2 and the general management area SC3 are communicably connected to one another via a local bus LB. Further, the devices provided in the sterile operation area SC2 and the devices provided in the other area SC4 are communicably connected to one another via a cloud network bus CB. The connection among the devices via the buses described above is merely an example, and is not limited to the connection method described above.

The control device 100 includes an operation control unit 101 and a detection control unit 102. The operation control unit 101 transmits a control signal to the robot device 300 so that the robot device 300 performs an operation related to a cell culture experiment. The detection control unit 102 transmits a control signal to the detection device 400 so that the detection device 400 detects a predetermined physical quantity or data or acquires detection data from the detection device 400.

Each of the terminal device 200 and the terminal device 201 includes an input unit and an output unit. The terminal device 200 and the terminal device 201 receive an input setting or an operation from a user such as a researcher through the input unit, and transmit, to the control device 100 or the detection device 400, a control signal or data corresponding to the input setting or the operation. Further, the terminal device 200 and the terminal device 201 acquire an output signal or data from the control device 100 or the detection device 400 and output the output signal or the data to the user by the output unit. The terminal device 200 and the terminal device 201 are both capable of allowing the user to perform an input setting and an operation related to operation control of the robot device 300. However, in general, the user performs the input setting and the operation related to the operation control of the robot device 300 using the terminal device 201. The terminal device 200 may be, for example, a personal computer, a tablet terminal, or a portable information terminal.

The robot device 300 includes various mechanisms, motors or actuators connected to the various mechanisms, and the like, and operates these based on the received control signal to perform operations of various processes related to the cell culture experiment. Further, the robot device 300 exchanges information with the cell culture apparatus 600, and delivers samples of cells to be cultured.

Main processes in the cell culture experiment are, for example, as follows.

First, there is a process called "medium replacement" . The cells use necessary nutrients from a culture solution and metabolize the nutrients. Therefore, a medium in which nutrients are reduced and an amount of metabolites is increased is discarded and replaced with a new medium. This operation (process) is referred to as the "medium replacement".

Second, there is a process called "cell subculture". It is necessary to collect a part of the cultured cells and transfer the cells to another new culture vessel. This process is also called subculture. Cells divide repeatedly during the culture and increase until the cells eventually fill up the culture vessel or the nutrients in the medium are depleted.

Third, there is a process called "cell seeding". Based on a measured number/concentration of the cells, an appropriate amount of "medium" is added for dilution to prepare a cell suspension, and a predetermined amount of the cell suspension is transferred to a new culture vessel, which is referred to as the "cell seeding". It can also be referred to as a process of setting the cell concentration to a target concentration and uniformly spreading the suspension in the culture vessel. The "cell suspension" is a suspension of cells (or microorganisms) being cultured in a liquid medium.

More specific operation contents in various processes of the cell culture experiment, that is, operations performed by the robot device 300, are as follows.

First, there is an operation called "pipetting". This refers to aspirating or discharging a liquid (cell suspension) with a pipette. In the cell culture, repeated aspirating and discharging of a cell suspension with a pipette can also serve to loosen cells in the cell suspension.

Secondly, there is an operation called "diffusion". This refers to an operation of uniformly diffusing a cell suspension on a bottom surface of a flask. Thirdly, there is an operation of "dispensing". This refers to an operation of accurately transferring a prescribed amount of a liquid such as a culture solution, a chemical solution, or a cell suspension to a plate or a tip with a pipette.

The detection device 400 detects information related to operation of the robot device 300. The detection device 400 includes an imaging unit 401 and a sensor unit 402.

The imaging unit 401 is provided in a laboratory and captures the operation performed by the robot device 300, for example, the operation of the robot device 300 corresponding to each process constituting the cell culture experiment. The imaging unit 401 is, for example, a digital video camera, and outputs video data obtained by the imaging.

The sensor unit 402 includes one or more force sensors. The one or more force sensors are provided in various mechanisms of the robot device 300, detect forces, pressures, stresses, twisting forces, and the like applied to the various mechanisms along with the operation of the robot device 300, and output data corresponding to detected physical quantities. The sensor unit 402 includes one or more environment sensors. The one or more environment sensors are provided in the laboratory, detect an environmental temperature, environmental humidity, an atmospheric pressure, a composition of an atmosphere, and the like in the laboratory, and output data corresponding to detected physical quantities.

The detection control unit 102 controls a time or a period for capturing images by the imaging unit 401, and cooperates with the operation control unit 101, if necessary, to synchronize timing of operations or processes.

The cell culture apparatus 600 includes a culture unit 601, a measurement unit 602, and a chemical solution installation unit 603.

The culture unit 601 includes an incubator or the like, and cultures the cells at a constant temperature and humidity. The measurement unit 602 measures a physical quantity representing a culture state of all or some of the cultured cells. The chemical solution installation unit 603 stores a culture medium as a nutrient for culturing the cells, a chemical solution used for measurement, a consumable to which a chemical solution is added, an instrument that aspirates and discharges a liquid such as a culture solution or a chemical solution, a consumable thereof, and the like.

The database device 500 has a data storage area such as a cloud system that stores various data, information, and the like transmitted from the terminal devices 200 and 201, the detection device 400, the recipe determination device 550, the cell culture apparatus 600, and the like via the control device 100. Data stored in the database device 500 includes, for example, the video data and environmental data obtained by the detection device 400, determination result data obtained by the recipe determination device 550, and history data of a determination recipe determined in the past. The data acquired by each of the above devices in a progress of the experiment and stored in the database device 500 is referred to as intermediate progress data.

The recipe determination device 550 sets a recipe to be applied to each of a plurality of samples of cells to be cultured. Further, the recipe determination device 550 stores priorities of verification for a plurality of types of environmental parameters that constitute the recipe in order to improve efficiency of the experiment. Top N environmental parameters in a priority order are called a determination recipe. The recipe determination device 550 refers to the intermediate progress data from the database device 500 in order to correct and update the determination recipe according to a predetermined algorithm.

The cell culture experiment is performed through the operation of the robot device 300. The robot device 300 performs the experiment in cooperation with another device or unit. For example, the robot device 300 temporarily transfers workpieces of cultured cells to the culture unit 601 and collects the cells, or passes the cells to the measurement unit 602 for measurement and then retrieves the cells and begins an operation, or takes the chemical solution from the chemical solution installation unit 603 and applies the chemical solution to the cultured cells.

The priority, the predetermined processes, and the like of the experiment performed by the robot device 300 are instructed by the operation control unit 101. The operation to be performed by the robot device 300 through the operation control unit 101 is determined in advance by the recipe determination device 550 based on a recipe set for the samples. The recipe determination device 550 selects a verification parameter that is an environmental parameter to be verified. Alternatively, sampling is prioritized, and the operation control unit 101 is instructed to perform the experiment according to the priority.

The recipe determination device 550 corrects the determination recipe based on a tendency analysis of a state of the cultured cell, which is obtained based on the intermediate progress data stored in the database device 500. The recipe determination device 550 corrects the past determination recipe using a result index stored in the database device 500. In the recipe determination device 550, in a case in which artificial intelligence finds, in the intermediate progress data, a causal relationship between the environmental parameter and an expected culture result, which is different from a causal relationship in the related art, the artificial intelligence improves a weight determination of the tendency analysis in the cell culture of the sample, which is performed based on the intermediate progress data, that is, a reference when a tendency is determined in the tendency analysis.

Here, main functions of the devices constituting the cell culture experiment system 10 according to Embodiment 1 are summarized as follows.

The control device 100 sets the recipe to be applied to each of the plurality of samples of the cells to be cultured. The control device 100 controls the operation of the robot device 300 such that the robot device 300 performs the culture experiment on each of the plurality of samples according to the set recipe. In the database device 500, the intermediate progress data for each of the sample is stored in a time series manner in the progress of one culture experiment in which a culture result of each of the plurality of samples is determined.

The recipe determination device 550 analyzes a tendency in the culture for each sample based on the intermediate progress data for each sample stored in the database device 500. The recipe determination device 550 calculates, for each sample, a posterior probability representing a degree of possibility that a value of each of the plurality of environmental parameters that constitute the set recipe causes the future successful culture of the sample, based on an analysis result of the tendency in the culture of the sample and a difference in the time-series intermediate progress data of the sample. The recipe determination device 550 determines one or more of the environmental parameters having a relatively high calculated posterior probability as a verification parameter. The recipe determination device 550 corrects the recipe for each sample by changing a parameter value of the determined verification parameter.

The control device 100 controls the operation of the robot device 300 such that the robot device 300 continues to perform the plurality of culture experiments according to the corrected recipes.

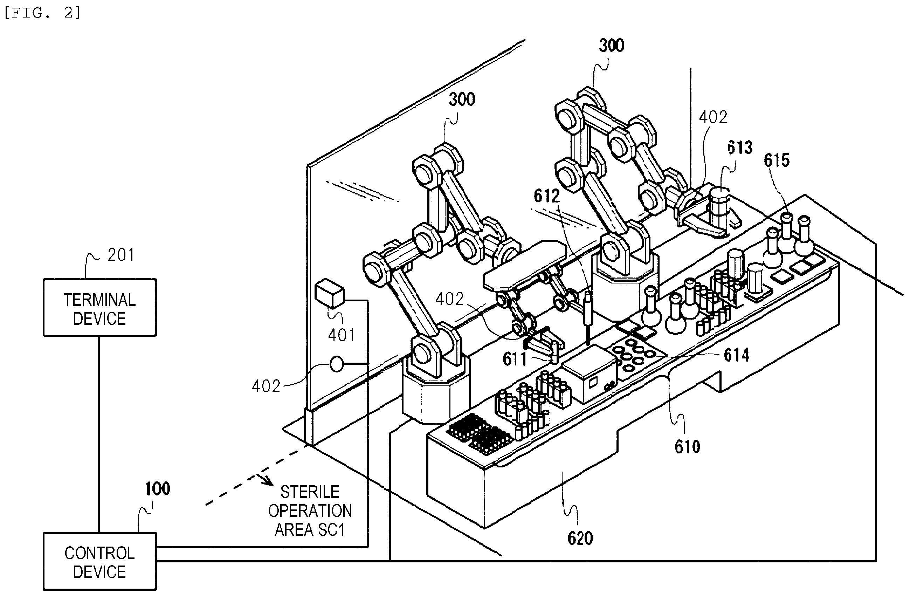

FIG. 2 is a diagram showing an appearance example of a main part of the cell culture experiment system according to Embodiment 1. As shown in FIG. 2, the control device 100, the terminal device 201, the robot device 300, and the detection device 400 are provided. Further, a table 620 is provided, and a plurality of processing target instruments 610 to be processed and operated by the robot device 300 are placed on the table 620. The instruments 610 include a test tube 611, a pipette 612, a vessel 613, a well plate 614, a flask 615, and the like. Further, each of the test tube 611, the pipette 612, and the vessel 613 in the instruments 610 is operated by the robot device 300. The robot device 300, the plurality of instruments 610, and the like are imaged by the imaging unit 401. Further, the environmental temperature, the environmental humidity, the atmospheric pressure, the composition of the atmosphere, and the like in the laboratory are detected by the environment sensor in the sensor unit 402. Further, the pressures, the twisting forces, the stresses, and the like generated in the various mechanisms constituting the robot device 300 are detected by the force sensor in the sensor unit 402.

The robot device 300 includes a base disposed on a floor. Further, the robot device 300 includes a rotatable joint connected to the base and an arm connected to the joint. A joint and an arm are further connected to a tip of the arm. A gripper that grips the instrument 610 is connected to a tip of the arm. The robot device 300 moves and operates the instrument 610 by the joint, the arm, the gripper, and the like. A video captured by the imaging unit 401 is displayed on the output unit of the terminal device 201 via the control device 100, for example. Furthermore, the robot device 300, the table 620, the instrument 610, and the like are disposed in the sterile operation area SC1. By disposing the instrument 610 or the like in the sterile operation area SC1, it is possible to prevent foreign matters or the like from being mixed into the sample or the like. Other devices such as a device that conveys the instrument 610 or the like may be disposed in the sterile operation area. In this example, the terminal device 201 is disposed in the sterile operation area SC2 different from the sterile operation area SC1, and can be operated by the user. The area where the terminal device 201 is disposed may be another area having a lower grade of cleanliness than the sterile operation area SC1, and is not limited to this example.

The control device 100, the database device 500, and the recipe determination device 550 can be implemented by using a dedicated or general-purpose computer such as a personal computer (PC) or a work station (WS), or an electronic device equipped with a computer. Further, the terminal device 201 can be implemented by using a dedicated or general-purpose computer such as a PC, a smartphone, a mobile phone, a tablet terminal, or a personal digital assistant (PDA), or an electronic device equipped with a computer.

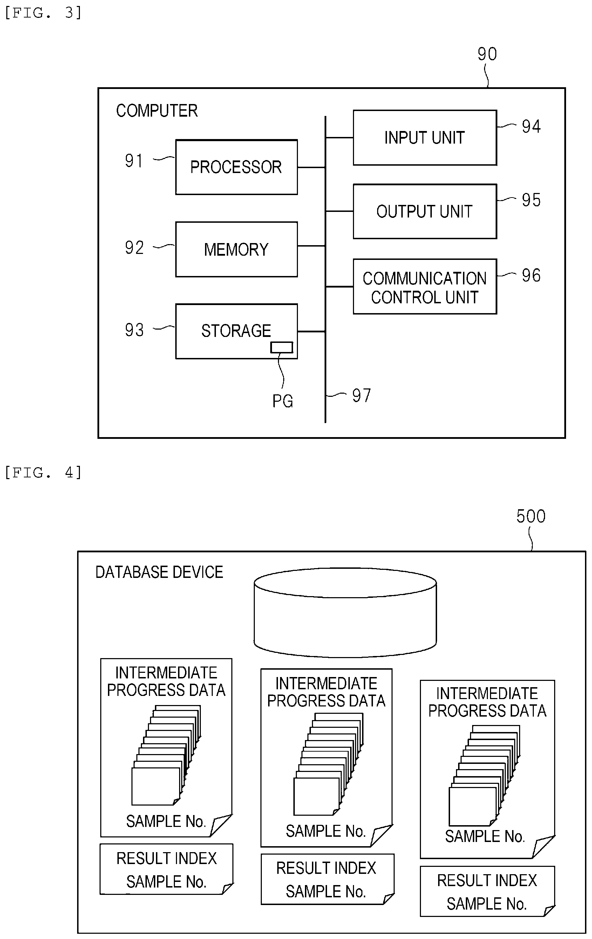

FIG. 3 is a diagram showing a configuration example of the computer. As shown in FIG. 3, a computer 90 includes a processor 91, a memory 92, a storage 93, an input unit 94, an output unit 95, a communication control unit 96, and a communication bus 97. The processor 91, the memory 92, the storage 93, the input unit 94, the output unit 95, and the communication control unit 96 are connected to one another via the communication bus 97 in a manner of being able to transmit and receive signals to and from one another. A predetermined program PG is stored in the storage 93. The computer 90 functions as, for example, the control device 100, the database device 500, or the recipe determination device 550 by the processor 91 reading the program PG and expanding and executing the program PG in the memory 92.

FIG. 4 is a diagram illustrating a configuration example of the data stored in the database device. As shown in FIG. 4, the database device 500 stores, for each of the sample of cells to be subjected to the culture experiment, a sample number (No.) assigned to the sample, intermediate progress data related to the sample, an experimental result index, and the like in association with each other.

<Flow of Processing in Cell Culture Experiment System>

Here, an example of a flow of processing in the cell culture experiment system 10 according to Embodiment 1 will be described.

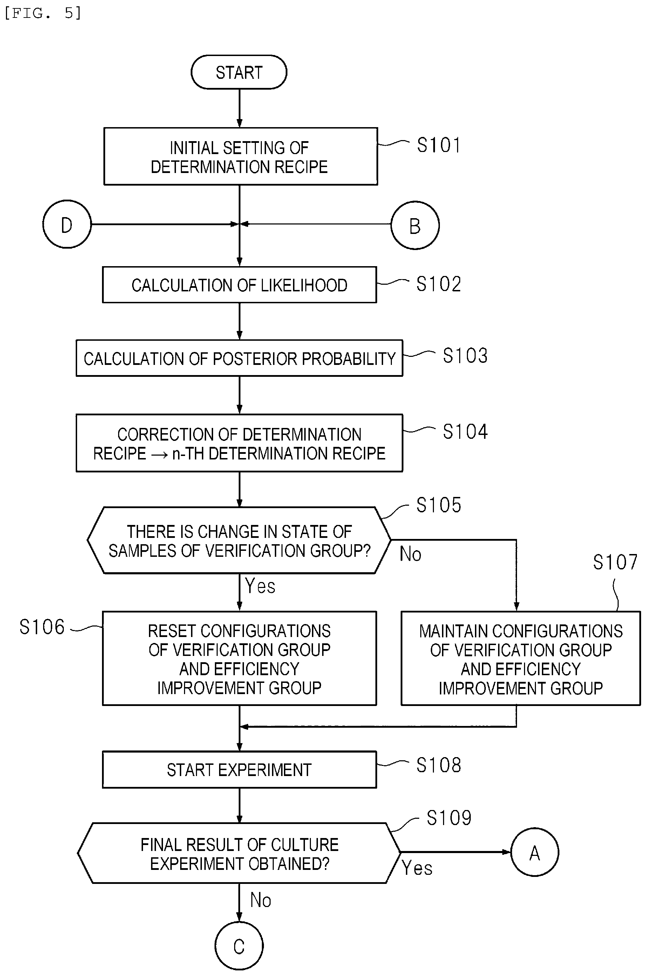

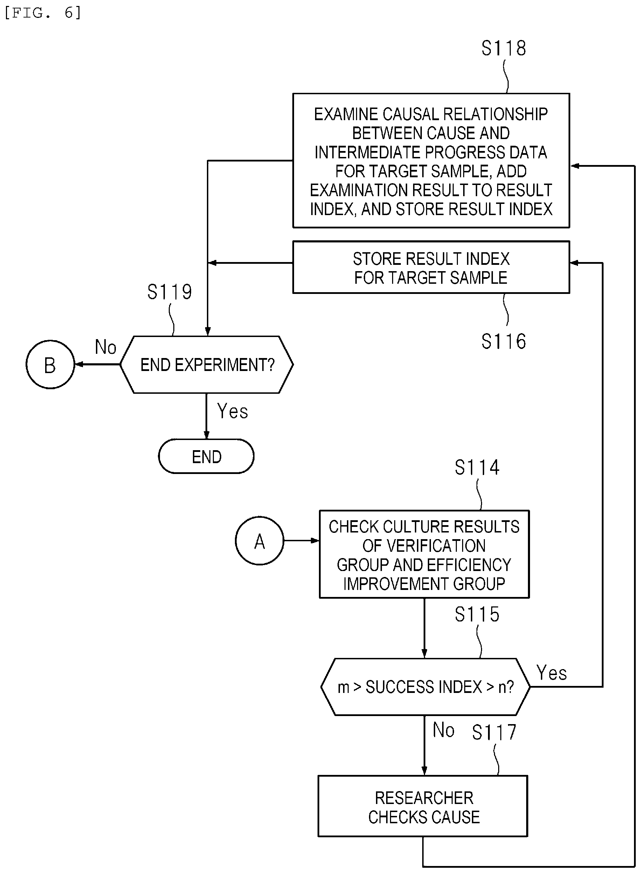

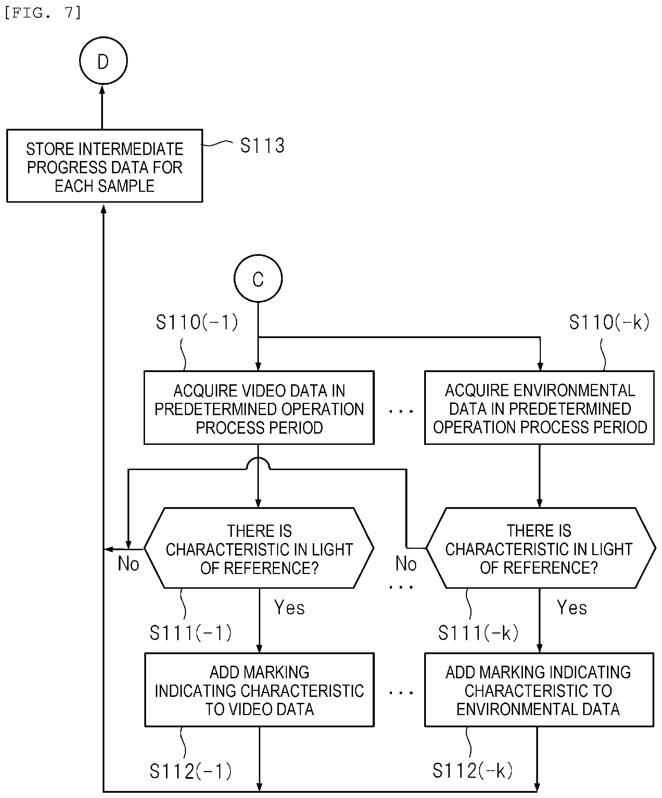

FIGS. 5 to 7 are diagrams showing examples of the flow of processing in the cell culture experiment system according to Embodiment 1.

As shown in FIG. 5, in step S101, an initial setting of the determination recipe is performed. Specifically, the user inputs, from the terminal device 201, information that defines the initial setting of a recipe to be set for each of a plurality of cell samples (hereinafter simply referred to as samples). The plurality of samples are divided into a group called the "verification group" and a group called the "efficiency improvement group". The terminal device 201 transmits the input information of the initial setting to the recipe determination device 550. The recipe determination device 550 stores, in a storage unit of the recipe determination device 550, recipe information of each of the samples belonging to the verification group and recipe information of each of the samples belonging to the efficiency improvement group based on the received information of the initial setting.

Next, in step S102, the likelihood is calculated. The "likelihood" corresponds to a "likelihood" used in the Bayes theorem. In step S102, specifically, the recipe determination device 550 accesses the database device 500 and determines whether there is intermediate progress data of the plurality of samples. In a case in which it is determined that there is no intermediate progress data, the recipe determination device 550 uses the artificial intelligence (AI) that has performed learning based on an experiment result of a previous cell culture to calculate a likelihood of a currently set recipe as an estimated value. On the other hand, when it is determined that there is intermediate progress data, the recipe determination device 550 reads out the intermediate progress data of the plurality of samples from the database device 500, and substitutes the read intermediate progress data into a predetermined calculation formula to calculate the likelihood for each sample.

Next, in step S103, a posterior probability is calculated. The "posterior probability" corresponds to a "posterior probability" used in the Bayes theorem, and here, represents a probability of successful culture in the future when the sample is cultured according to the recipe. More strictly, the posterior probability is a success rate of the cell culture for each environmental parameter to be verified (for example, a score between 0% and 100%). In step S103, specifically, the recipe determination device 550 calculates, based on a prior probability set in advance according to the recipe for each of the plurality of samples and the calculated likelihood using the artificial intelligence, the posterior probability for each environmental parameter in the recipe for each sample by the Bayes theorem.

Next, in step S104, an n-th determination recipe is corrected. As described above, the "determination recipe" refers to a recipe for a determination, which is set for each sample. The recipe is repeatedly rewritten and corrected a plurality of times based on the intermediate progress data obtained in the progress of the culture experiment performed according to the recipe. Therefore, the recipe that is currently determined is called the determination recipe.

In step S104, specifically, the recipe determination device 550 rewrites the determination recipe for each of the samples in the verification group and the efficiency improvement group by the Bayes theorem based on the posterior probability calculated for the sample using the artificial intelligence.

The determination recipe includes a list of a plurality of types of environmental parameters to be verified and a priority for each of the environmental parameters. The priority is determined from a result of the tendency analysis of the cell culture based on the intermediate progress data. The tendency analysis of the intermediate progress data is to analyze a tendency of intermediate progress of the cell culture based on the intermediate progress data, or to know a state of acceleration, stagnation, canceration, or the like of cell growth (proliferation). The cell culture often takes a long time. In the progress of the culture, growth may gradually stagnate, or at some time point, the cells may suddenly become cancerous, or in some cases, the stagnant growth may begin to accelerate. The tendency analysis is to analyze a tendency in an intermediate progress of such cell culture.

Here, the recipe determination device 550 uses the artificial intelligence to analyze the intermediate progress data, calculates a degree to which each environmental parameter is likely to affect a success or failure of the cell culture, that is, a priority, and assigns that priority to each environmental parameter. Further, the top N environmental parameters in the priority order are set as verification parameters, which are parameters to be verified, and parameter values of the verification parameters are assigned and set among the recipes of the plurality of samples. That is, the recipe determination device 550 extracts the verification parameter from the analysis result of the intermediate progress data, and assigns and sets a parameter value for the environmental parameter corresponding to the extracted verification parameter among the environmental parameters of the recipes of the plurality of samples.

That is, instead of verifying all patterns in a combination of a large number of environmental parameter values, a pattern determined to have a high priority based on the recipe determination is extracted, and a recipe of the pattern is verified. That is, a content of a culture process for each sample is corrected. The priority is the posterior probability and is derived by the artificial intelligence.

The artificial intelligence used here has performed the learning, or continues to perform the learning, to prioritize the verification parameters such that a sample that is set with a combination of a type of the environmental parameter considered to be highly likely to contribute to successful cell culture and a value of the parameter has a higher success rate, and conversely, a sample that is set with a combination of a type of the environmental parameter considered to be highly likely to contribute to failed cell culture and a value of the parameter has a higher failure rate (lower success rate). That is, the artificial intelligence can make rational determination to reduce the number of verification parameters, for example, to "verify only top 20% of the verification parameters" in the priority order.

The rewriting, that is, the correction of the determination recipe is performed such that the posterior probability becomes higher for the sample belonging to the verification group and the posterior probability becomes lower for the sample belonging to the efficiency improvement group. The "n-th" of the n-th determination recipe starts from n = 1 and increments to n = 2, n = 3, ..., every time the value is rewritten.

Next, in step S105, for the sample belonging to the verification group, a determination is made as to whether there is a change in a state of the cell. That is, a determination is made as to whether to reset configurations of the samples in the verification group and the efficiency improvement group. Specifically, the recipe determination device 550 determines whether to reset the configurations of the samples belonging to the verification group and the efficiency improvement group such that the verification group becomes a group having a higher priority, based on whether there is a change in the verification group with a predetermined sample population.

For example, the recipe determination device 550 determines whether there is a change in the state of the cell based on information such as the number (density) of cells, a proliferation rate of the cells, and the presence or absence of canceration of the cells in the intermediate progress data for the sample belonging to the verification group. Here, when it is determined that there is a change in the state of the cell (S105: Yes), the processing step proceeds to step S106 to reset the configurations of the verification group and the efficiency improvement group. On the other hand, when it is determined that there is no change in the state of the cell (S105: No), the processing step proceeds to step S107 to maintain the configurations of the samples in the verification group and the efficiency improvement group.

In step S106, the configurations of the samples in the verification group and the efficiency improvement group are reset. Specifically, the recipe determination device 550 resets the configuration of the samples belonging to the verification group and the configuration of the samples belonging to the efficiency improvement group based on the change in the state of the cells using the artificial intelligence. More specifically, as described above, the artificial intelligence resets the configurations of the samples in the verification group and the efficiency improvement group such that a group of samples set with the combination of the type of the environmental parameter considered to be highly likely to contribute to the successful cell culture and the value of the parameter has a higher culture success rate, and conversely, a group of samples set with the combination of the type of the environmental parameter considered to be highly likely to contribute to the failed cell culture and the value of the parameter has a lower culture success rate (higher failure rate). Thereafter, the processing step proceeds to step S108.

In step S107, the configurations of the samples in the verification group and the efficiency improvement group are maintained. Specifically, the recipe determination device 550 maintains a current state without changing the configurations of the samples belonging to respective verification group and efficiency improvement group at the current time. Thereafter, the processing step proceeds to step S108.

In step S108, an experiment is started. Specifically, the operation control unit 101 starts a process of transmitting the control signal to the robot device 300 such that the culture experiment is started in descending order of set priority for each of the samples belonging to the verification group and the efficiency improvement group, and the culture experiment is performed according to the recipe set for each sample. Further, the operation control unit 101 transmits a control signal to the units of the cell culture apparatus 600 as necessary such that preparation, a condition setting, and the like necessary for the experiment are performed on the cell culture apparatus 600. Further, the detection control unit 102 transmits the control signal to the detection device 400 as necessary such that the detection device 400 performs the imaging by the imaging unit 401 or acquisition of data by the sensor unit 402.

In step S109, it is determined whether a final result of the culture experiment is obtained. Specifically, the control device 100 accesses the database device 500, determines whether information related to the final result of the culture experiment currently performed is stored, and determines whether the final result of the culture experiment currently performed is obtained based on the determination result. Here, in a case in which the control device 100 determines that the final result is obtained (S109: Yes), the processing step proceeds to step S114 so that checking of the culture result is performed. On the other hand, in a case in which the control device 100 determines that the final result is not obtained (S109: No), the processing step proceeds to step S110 such that the intermediate progress data is stored in the database device 500.

In step S110, the intermediate progress data in a predetermined operation process period of the robot device is acquired. Specifically, the detection device 400 acquires video data obtained by recording the operation of the robot device 300 (S110-1) or acquires the environmental data such as a temperature, humidity, and an atmospheric pressure in the laboratory (S110-k) for an operation process currently performed among a plurality of predetermined operation processes of the robot device 300. Further, the detection device 400 acquires, from the recipe determination device 550 or the control device 100, operation data that is control data defining an operation in each operation process of the robot device 300.

In step S111, the recipe determination device 550 determines whether the acquired intermediate progress data has a characteristic in light of a reference. Specifically, the recipe determination device 550 stores reference information for each type of the intermediate progress data, and determines whether the characteristic is present by comparing each acquired intermediate progress data with the reference. The determination here may be a threshold determination of an index value obtained based on the intermediate progress data, or may be a determination based on the analysis result of the intermediate progress data which is obtained by the artificial intelligence.

For example, the recipe determination device 550 determines whether there is a characteristic operation different from an assumed operation in the video data in which the operation of the robot device 300 is recorded by using the artificial intelligence or by evaluation and the input of the user (S111-1). Further, for example, the recipe determination device 550 determines whether there is a characteristic that the temperature, the humidity, the atmospheric pressure, or the like is separated from an assumed reference value by a predetermined level or more in the environmental data by using the artificial intelligence or by the evaluation or the input of the user (S111-k). Furthermore, for example, the recipe determination device 550 determines whether there is characteristic control in the operation data of the robot device 300 by using the artificial intelligence or by the evaluation or the input of the user.

The processing step proceeds to step S112 to process the environmental parameter determined to have a characteristic in the determination in step S111. On the other hand, the processing step proceeds to step S113 to process the environmental parameter determined to have no characteristic in the determination in step S111.

In step S112, a marking indicating a characteristic is added to the intermediate progress data. Specifically, the recipe determination device 550 adds a marking indicating a portion determined to have a characteristic in the intermediate progress data. For example, the recipe determination device 550 adds, to the video data, a marking associated with a start time point of a time zone in which an operation determined to have a characteristic is performed in the video data (S112-1). Further, for example, the recipe determination device 550 adds, to the environmental data, a marking associated with a start time point of a time zone in which a change determined to have a characteristic occurs in the environmental data (S112-k). When the addition of the marking is completed, the processing step proceeds to step S113.

In step S113, the intermediate progress data is stored for each sample. Specifically, the control device 100 stores the intermediate progress data in the culture experiment of each sample in a storage area of the database device 500. In the database device 500, a folder is prepared for each sample number, and the intermediate progress data of each sample is stored in the folder with the sample number of that sample. That is, the intermediate progress data corresponding to each operation process of the robot device 300 in the culture experiment for each sample is stored in the folder prepared for each sample. When the storage of the intermediate progress data is completed, the processing step returns to step S102.

In step S114, the final culture result is checked. Specifically, the control device 100 outputs the final culture result of each sample in the verification group and the efficiency improvement group to the user, for example by displaying the final culture result on a screen of a display unit of the terminal device 201. The user such as a researcher inputs a success index of the culture experiment for each sample with reference to the culture result. When the input is completed, the processing step proceeds to step S115.

In step S115, whether the success index is within a preset range is determined. Specifically, the recipe determination device 550 determines, for each sample, whether a value of the success index is within a range of m to n that is the preset range.

In this determination, for a sample of which the success index is determined to be within the set range (S115: Yes), the recipe determination device 550 sets a result that the success index of the sample is within the set range as a result index, and stores the result index in the folder of the target sample of the database device 500 (S116).

On the other hand, in this determination, for a sample of which the success index is determined to be not within the set range, that is, the success index is outside the set range (S115: No), the recipe determination device 550 determines a result that the success index of the sample is outside the set range as the result index. Further, the user such as a researcher checks a cause of the result and inputs information representing the cause through the terminal device 201 (S117). For example, the user extracts a difference causing an extremely good or bad result from the intermediate progress data, adds an extraction result to the result index by operating the terminal device 201, and stores the result index in the database device 500. Further, for example, the recipe determination device 550 examines a causal relationship between the cause and the intermediate progress data using the artificial intelligence for the sample, adds the examination result to the result index, and stores the result index in the folder of a target sample of the database device 500 (S118).

When the processing for each sample in steps S115 to S118 is completed, the processing step proceeds to step S119.

In step S119, whether to end the experiment is determined. Specifically, the control device 100 determines whether to end the culture experiment based on information input by the user through the terminal device 201 or based on information representing a preset experimental design. Here, when the control device 100 determines to end the culture experiment (S119: Yes), a process related to the culture experiment ends. On the other hand, the control device 100 determines not to end the culture experiment, that is, to continue the culture experiment (S119: No), the processing step returns to step S102, and the process related to the culture experiment is continued.

As described above, when the process is performed according to the processing flows shown in FIGS. 5 to 7, the intermediate progress data and an evaluation result thereof are stored in the database device 500 for each operation process of the robot device 300 and for each sample. Further, based on the stored intermediate progress data, the recipes of the samples in the verification group and the efficiency improvement group, that is, parameter sets of the intermediate environmental parameters are sequentially corrected. As a result, samples for which recipes have a high probability of success of culture are collected in the verification group, and samples for which recipes have a low probability of success of culture are collected in the efficiency improvement group.

The recipes of the samples in the verification group converged in this way reveal the types of environmental parameters and the conditions thereof that are likely to lead to the successful culture. On the other hand, the recipes of the samples in the efficiency improvement group converged in this way reveal the types of environmental parameters and the conditions thereof that are highly likely to lead to the failed culture, or the types of environmental parameters that are highly likely not to affect the success or failure of culture.

A definition of the successful cell culture varies depending on the user of the system, and there are no specific conditions. The successful cell culture can be defined, for example, as normality and the number of cells after the culture process is completed meeting predetermined conditions. Further, the successful cell culture can also be defined as the normality and the number of cells meeting predetermined conditions and the cells being successfully frozen after the culture process is completed. The user of the system may determine the definition of the successful culture and intuitively add the success index (score) as accompanying data of the result index for each sample.

In Embodiment 1, one of the most characteristic portions is that the video data, the operation data, data detected by a sensor, and the like, which are related to the operation of the robot device 300 in the progress of the experiment, are stored as the intermediate progress data. Previous research has revealed that the success or failure of the cell culture is affected by operations such as how the vessel containing the cells is handled, how the chemical solution or cell liquid is suctioned or discharged with a pipette, and what kind of impact is given to the cells. Therefore, storing the data related to the operation of the robot device 300 in relation to these operations as the intermediate progress data is extremely important in searching for an appropriate recipe for the successful culture, that is, the types of environmental parameters and the conditions thereof.

That is, for example, information related to the operation of the robot device 300 corrected for each sample as intended by the user is considered as the intermediate progress data. The information related to the operation of the robot device 300 is, for example, information such as a trajectory of movement of an instrument to be handled, a speed of the movement of the instrument, acceleration of the movement of the instrument, a posture of the instrument, a pressure applied to an object by the instrument, and a twisting force applied to the object by the instrument. These pieces of information are different from uniform data that is determined in advance through teaching. Hereinafter, specific examples of the intermediate progress data will be described.

<Specific Example 1 of Intermediate Progress Data>

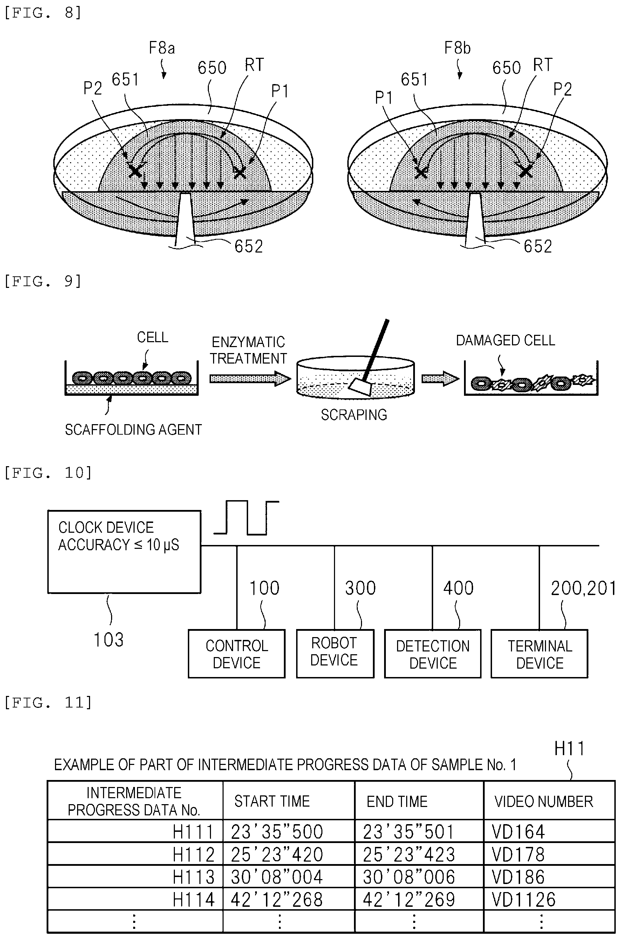

FIG. 8 is a diagram showing an example of the operation of the robot device according to the operation data. As the operation data stored as the intermediate progress data, for example, as shown in FIG. 8, data related to the operation of the robot device 300 when a liquid 651 which is a chemical solution or a cell solution in a petri dish 650 is aspirated once by a pipette 652 and discharged is considered. Further, the operation data includes, for example, a discharge start position P1, a trajectory RT of the pipette during the discharge, a discharge end position P2, and data representing an operation corresponding to each condition such as natural cell mass separation by applying the pipette 652 to a wall of the petri dish 650, quiet mixing of the liquid 651 spilling from the right to the left (F8a in FIG. 8), and natural fluid rotation of the liquid 651 spilling from the left to the right (F8b in FIG. 8).

<Specific Example 2 of Intermediate Progress Data>

The control device 100 operates the robot device 300 based on the created operation data, that is, the scenario. The detection device 400 monitors the operation of the robot device 300 and outputs the operation to the user from the terminal device 201. When the robot device 300 is operating based on the scenario, the control device 100 receives an input of correction to the operation from the user via the terminal device 201 and corrects the scenario.

FIG. 9 is a diagram showing a process of peeling adherent cells in a cell culture process. In the process of peeling the adherent cells as shown in FIG. 9, many cells can be properly collected by a scraping operation using a scraping jig, but some cells subjected to an impact during the scraping are damaged and die.

Here, for example, it is assumed that the detection device 400 performs detection on video data of a scraping operation on cells during a certain time zone. A speed of the scraping operation is approximately 10 cm/sec when the scraping is performed by a human hand. If the video of the scraping operation is important as the intermediate progress data, it is naturally important to ensure that the scraping operation occurs over a distance of approximately 100 $\mu$m, from a time when the scraping operation begins to have a physical effect on the cell in the vicinity until the hand comes into contact with the cell and some kind of event occurs, that is, a distance approximately equal to or greater than a size of the cell.

In the case of the above example, by a simple calculation, a time required when the scraping is performed by the robot device 300 over a distance of approximately 100 $\mu$m is at most 1 ms. Therefore, there is a need for time consistency that can ensure that a series of operations have been performed within a short time period of 1 ms among a plurality of devices.

FIG. 10 is a diagram showing a state in which a plurality of devices are operated by a common clock signal. In general, for example, as shown in FIG. 10, the control device 100, the robot device 300, the detection device 400, and the terminal device 200 and 201 are controlled by a clock signal common to each device which is output from a clock device 103 having accuracy on the order of microseconds. Therefore, it is possible to ensure the time consistency among the devices as required in the above example. That is, it is possible to align the operations of the devices for an event that occurs within 1 millisecond, and it is possible to ensure the intermediate progress data such as "a series of operations from xx minutes xx seconds 500 milliseconds to xx minutes xx seconds 501 milliseconds". A time error representing the accuracy of the common clock signal output from the clock device 103, for example, an error of a time interval of a rising edge is preferably 10 $\mu$S (microseconds) or less.

FIG. 11 is a diagram showing an example of a plurality of pieces of video data that are a part of the intermediate progress data. In the case of the above example, for each sample, for example, as shown in FIG. 11, the plurality of pieces of video data are acquired as the intermediate progress data. The example of FIG. 11 shows a state in which the plurality of pieces of video data are acquired as intermediate progress data H11 for a sample having a sample No. 1. Each piece of video data constituting the intermediate progress data H11 is, for example, data in which intermediate progress data No., a start time, an end time, and a video number are associated with one another.

If there is no time consistency among the devices, for example, it is impossible to determine which time zone the detected operation is caused by operating the robot device 300, there is no consistency among the devices, and the data becomes meaningless as the intermediate progress data. In this way, it is possible to ensure a series of intermediate progress data obtained by cooperation of the devices using the time consistency. Depending on an application in an actual research site, verification of more microscopic operations may be required, and in this case, it goes without saying that even more precise time consistency is required.

<Specific Example 3 of Intermediate Progress Data>

Specific Example 3 of the intermediate progress data is an example in which measurement data of a pressure generated in a culture instrument which is acquired by the force sensor is the intermediate progress data.

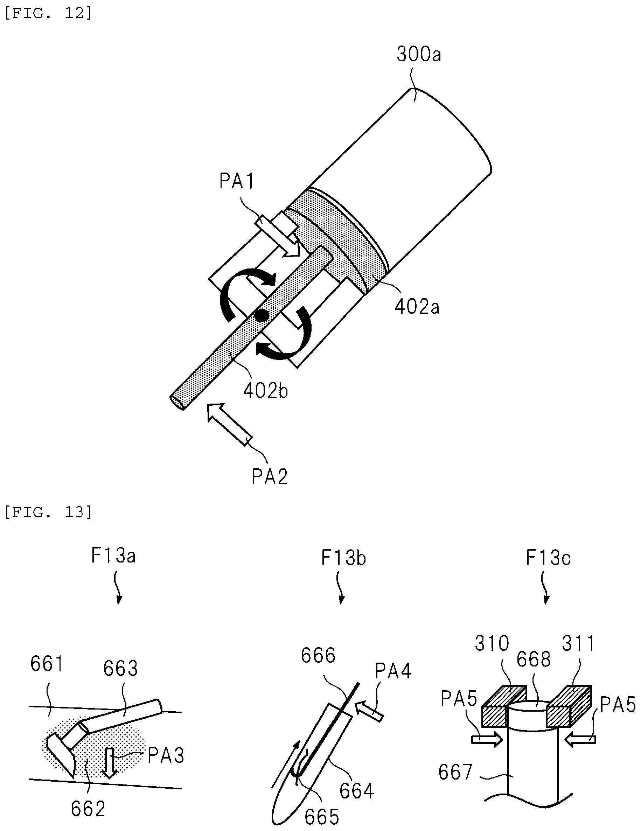

FIG. 12 is a diagram showing the force sensor as an example of the sensor unit of the detection device. For example, as shown in FIG. 12, a force sensor 402a has a configuration in which a lever is applied, and is a sensor that detects a pressure PA1 applied to an object that is in contact with an operating instrument 402b held by a part 300a of the robot device by detecting a pressure PA2 that is generated according to a principle of action and reaction. The force sensor 402a is formed, for example, by combining a plurality of sensors called strain sensors that convert a pressure into an electrical signal, and can quantitatively grasp the pressure.

FIG. 13 is a diagram showing a specific case related to the contact of the object. A first case relates to a process of peeling the adherent cells. More specifically, the first case relates to a process of peeling the adherent cells on the bottom surface 661 of the petri dish by moving cells 662 that are cultured while adhering to a bottom surface 661 of the petri dish while pressing a scraping instrument 663 against the bottom surface 661 of the petri dish, for example, as shown in FIG. 13 and a diagram shown by a symbol F13a. It is important to press a scraping jig while applying a predetermined pressure to the bottom surface 661 of the petri dish so as not to damage the cells 662 and to ensure that the cells are sufficiently peeled. Therefore, a degree of a pressure PA3 applied to the bottom surface 661 of the petri dish, that is, a measured value of the pressure PA3, can be used as the intermediate progress data.

A second case relates to a process of taking out a specimen. More specifically, the second case relates to a process of taking out a specimen 665 from a test tube 664 by pressing an instrument 666 against an inner wall of the test tube 664 and pulling the instrument 666 out, for example, as shown in FIG. 13 and a diagram shown by a symbol F13b. In this case, a degree of a pressure PA4 applied to an inner wall surface of the test tube 664, that is, a measured value of the pressure PA4, can be used as the intermediate progress data.

A third case relates to a process of opening and closing a test tube lid. More specifically, the third case relates to a process in which gripping mechanisms 310 and 311 of the robot device grip a lid 668 and open and close the lid 668 in an operation of rotating and opening and closing the lid 668 of the test tube 667, for example, as shown in FIG. 13 and a diagram shown by a symbol F13c. In this case, a degree of a pressure PA5 applied to the lid 668 of the test tube 667 when the lid 668 is opened or closed, that is, a measured value of the pressure PA5, can be used as the intermediate progress data.

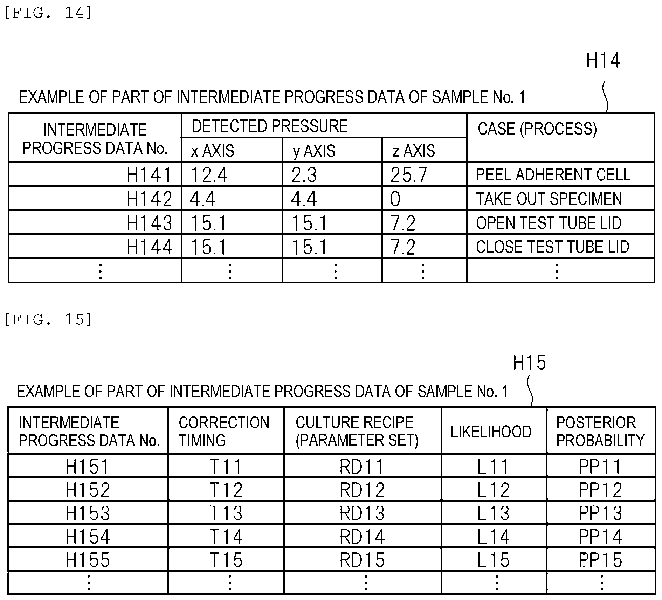

FIG. 14 is a diagram showing an example of data of a pressure applied to the object in the processes, each of which is a part of the intermediate progress data. The force sensor can measure a plurality of parameters such as a pressure in an axial direction or a pressure in a torque direction. For each sample, for example, as shown in FIG. 14, data of detected pressures on an x axis, a y axis, and a z axis in each process is acquired as the intermediate progress data. The example in FIG. 14 shows a state in which a plurality of pieces of detected pressure data are acquired as intermediate progress data H14 for the sample with the sample No. 1. The individual detected pressure data constituting the intermediate progress data H14 is, for example, data in which intermediate progress data No., the detected pressure of the x axis, the detected pressure of the y axis, the detected pressure of the z axis, and the case (process) are associated with one another. The cases (processes) include the adherent cell peeling, the taking of the specimen, the opening of the test tube lid, and the closing of the test tube lid.

In this way, with respect to the operation of the robot device 300 in each process of the culture experiment, it is possible to ensure data on the type of force sense used for performing the operation as the intermediate progress data.

<Another Specific Example of Intermediate Progress Data>

Of course, data that can be the intermediate progress data is not limited to the above, but also includes, for example, the following. Examples thereof include an angle at which the pipette is held, a speed at which the flanger of the pipette is pushed and pulled, a timing at which the flanger is pushed and pulled, an acceleration at the time of movement of the flanger, a condition related to an operation of a tip of the pipette from contacting the tip with a liquid to removing the tip from the liquid, a speed at which the petri dish is moved, an inclination of the petri dish at the time of movement of the petri dish, and an opening and closing speed of a door of an incubator. These can be the intermediate progress data, but at the same time, these can also be said to be the environmental parameters constituting the recipe to be verified.

FIG. 15 is a diagram illustrating an example of a part of the intermediate progress data. As shown in FIG. 15, as a part of the intermediate progress data, for example, intermediate progress data No., a correction timing of a culture recipe, a corrected culture recipe, a likelihood, and a posterior probability are stored in association with one another.

<Example of Effect of Embodiment 1>

As described above, according to Embodiment 1, in the cell culture experiment in which the posterior result is not obtained or it takes time to find the posterior result, a verification priority of the environmental parameter is determined based on a first determination recipe by the artificial intelligence that has learned the relationship between the setting of the environmental parameter and the culture result based on a large number of past experiment results. The first determination recipe is ensured with the intermediate progress data as additional information, and the first determination recipe is corrected by the intermediate progress data. Further, the corrected first determination recipe is applied to a next similar determination recipe. As described above, by utilizing the recipe sequentially corrected and updated in the progress of the culture experiment, it is possible to estimate the success or failure of the culture or the environmental parameter related to the successful culture and the condition thereof before the final result of the culture experiment is obtained.

Further, according to Embodiment 1, when the difference occurs in the cell culture state due to the change in the recipe, the difference in the intermediate progress data previously attached to that recipe is extracted, and the experiment result is predicted when the difference is present in the intermediate progress data. Accordingly, it is possible to shorten the experiment period, eliminate unnecessary experiments, and find causal information collection.

It is assumed that a cell culture experiment system performed by a robot without correcting a determined scenario is used for searching for some appropriate values among a large number of environmental parameters constituting the recipe of the cell culture. The environmental parameters to be searched for are, for example, a drug concentration and a drug addition time in the process 1, a drug treatment time, a cell peeling amount, and a cell peeling strength in the process 2, and a drug concentration and a drug addition time in the process 3. A timing of changing the environmental parameter to be searched for is after the end of the experiment, and the environmental parameter to be searched for can be automatically changed. However, when there are many environmental parameters, it takes a long time such as four months until all the experiments are completed.

It is better if the number of environmental parameters to be searched for is determined in advance by a person, but the vast majority of environmental parameters are not determined in advance. Therefore, with such a system, it is necessary to repeatedly perform the culture experiment by varying all the environmental parameters, feed back results, and then perform another culture experiment, which takes an extremely long time. That is, the search is practically difficult.

On the other hand, in the cell culture experiment system according to Embodiment 1, the intermediate progress data is analyzed, and the degree (priority) to which each environmental parameter is likely to affect the success or failure of the cell culture is calculated, and that degree is assigned to each parameter. Further, the top N parameters are set as the verification parameters, and the values of the verification parameters are set among the plurality of samples. That is, the content of the culture step for each sample is corrected. The degree is the posterior probability and is derived by the artificial intelligence.

In a case in which an environmental parameter indicating an extreme value of the posterior probability (outside a range of a singular point: 20% to 80%) is found, since there is a possibility that equipment may be missing or an accident may occur during the experiment, humans intervene to identify a causal relationship and add the identified cause to the intermediate progress data.

(Embodiment 2)

A cell culture experiment method as an example of an embodiment of the invention will be described. The cell culture experiment method is as follows.

A recipe determination device sets a recipe to be applied to each of a plurality of samples of a cell to be cultured. A control device controls an operation of a robot device such that the robot device performs a culture experiment on each of the plurality of samples according to the set recipe. A database device stores intermediate progress data for each of the samples in a time series manner in a progress of one culture experiment in which a culture result of each of the plurality of samples is determined. The recipe determination device analyzes a tendency in culture for each sample based on the intermediate progress data for each sample stored in the database device, and calculates, for each sample, a posterior probability representing a degree of possibility that a parameter value of each of a plurality of environmental parameters that constitute the set recipe causes future successful culture of the sample, based on an analysis result of the tendency in the culture of the sample and a difference in the time-series intermediate progress data of the sample. The recipe determination device determines one or more of the environmental parameters having a relatively high calculated posterior probability as a verification parameter, and corrects the recipe for each sample by changing a parameter value of the determined verification parameter. The control device controls the operation of the robot device such that the robot device continues to perform a plurality of culture experiments according to the corrected recipe.

According to such a cell culture experiment method, the same effect as that of the cell culture experiment system according to Embodiment 1 can be obtained, that is, it is possible to determine and implement a next recipe to be determined without waiting for an actual experiment result, and it is possible to shorten a period until an appropriate environmental parameter set is obtained.

(Embodiment 3)

A program as an example of an embodiment of the invention will be described. The program is as follows.

A program causing one or more computers or processors to function as:

a recipe determination device configured to set a recipe to be applied to each of a plurality of samples of a cell to be cultured;
a control device configured to control an operation of a robot device such that the robot device performs a culture experiment on each of the plurality of samples according to the set recipe; and
a database device configured to store intermediate progress data for each of the samples in a time series manner in a progress of one culture experiment in which a culture result of each of the plurality of samples is determined, in which the recipe determination device

analyzes a tendency in culture for each sample based on the intermediate progress data for each sample stored in the database device,
calculates, for each sample, a posterior probability representing a degree of possibility that a value of each of a plurality of environmental parameters that constitute the set recipe causes future successful culture of the sample, based on an analysis result of the tendency in the culture of the sample and a difference in the time-series

# EP 4 779 010 A1

intermediate progress data of the sample,

determines one or more of the environmental parameters having a relatively high calculated posterior probability as a verification parameter, and

corrects the recipe for each sample by changing a parameter value of the determined verification parameter, and

the control device controls the operation of the robot device such that the robot device continues to perform the plurality of culture experiments according to the corrected recipes.

Further, a tangible computer-readable non-transitory storage medium recording such a program is also an example of the embodiment of the invention.

According to such a program or storage medium, by reading and executing the program by one or more computers or processors, it is possible to obtain the same effects as those of the cell culture experiment system according to Embodiment 1, that is, it is possible to determine and execute a next recipe to be determined without waiting for an actual experiment result, and it is possible to shorten a period until an appropriate environmental parameter set is obtained.

As described above, according to this embodiment, in the cell culture experiment, even in the middle of the experiment (round), the tendency of the culture of each sample is analyzed based on the intermediate progress data obtained so far. Further, the system estimates a probability of success of the culture as the posterior probability based on the analysis result, regards the estimated posterior probability as an actually obtained experiment result, inputs the estimated posterior probability to a Bayesian estimation formula, and corrects the recipe (including parameters related to the operation of the robot) of each sample. By such a process of the system, it is possible to determine and execute the next recipe to be determined without waiting for the actual experiment result, and it is possible to shorten a period until an appropriate environmental parameter set is obtained.

The invention is not limited to the embodiments described above and includes various modifications. For example, the embodiments described above has been described in detail in order to facilitate understanding of the invention, and are not necessarily limited to those including all the configurations described above. Further, a part of the configuration of one embodiment can be replaced with the configuration of another embodiment, the configuration of one embodiment can be added to the configuration of another embodiment, and a part or all of the configurations, functions, processing units, processing methods, and the like described above may be implemented by hardware, for example, by designing these as an integrated circuit. The above configurations, functions, or the like may be implemented by software by a processor interpreting and executing a program for implementing each function. Information such as a program, a table, and a file for implementing each function can be stored in a recording device such as a memory, a hard disk, and a solid state drive (SSD), or in a recording medium such as an IC card, an SD card, and a DVD.

Reference Signs List

10 cell culture experiment system
100 control device
101 operation control unit
102 detection control unit
200, 201 terminal device
300 robot device
400 detection device
401 imaging unit
402 sensor unit
500 database device
550 recipe determination device
600 cell culture apparatus
601 culture unit
602 measurement unit
603 chemical solution installation unit
661 bottom surface of petri dish

## Claims

1.  A cell culture experiment system comprising:

a recipe determination device configured to set a recipe to be applied to each of a plurality of samples of a cell to be cultured;

a robot device;

a control device configured to control an operation of the robot device such that the robot device performs a culture experiment on each of the plurality of samples according to the set recipe; and

a database device configured to store intermediate progress data for each of the samples in a time series manner in a progress of one culture experiment in which a culture result of each of the plurality of samples is determined, wherein

the recipe determination device

analyzes a tendency in culture for each sample based on the intermediate progress data for each sample stored in the database device,

calculates, for each sample, a posterior probability representing a degree of possibility that a value of each of a plurality of environmental parameters that constitute the set recipe causes future successful culture of the sample, based on an analysis result of the tendency in the culture of the sample and a difference in the time-series intermediate progress data of the sample,

determines one or more of the environmental parameters having a relatively high calculated posterior probability as a verification parameter, and

corrects the recipe for each sample by changing a parameter value of the determined verification parameter, and

the control device controls the operation of the robot device such that the robot device continues to perform the culture experiment on each of the plurality of samples according to the corrected recipe.

2. The cell culture experiment system according to claim 1, further comprising:

a detection device configured to detect information on the operation of the robot device, wherein the intermediate progress data includes data representing the detected operation of the robot device.

3. The cell culture experiment system according to claim 2, wherein the data representing the operation of the robot device includes data capable of specifying at least one of a trajectory of movement of an instrument handled by the robot device, a speed of the movement of the instrument, an acceleration of the movement of the instrument, and a posture of the instrument.

4. The cell culture experiment system according to claim 3, wherein

the cell is an adherent cell, and
the operation of the robot device includes a process of peeling the adherent cell, a process of taking out a specimen from a vessel, or a process of opening and closing a lid of a test tube.

5. The cell culture experiment system according to claim 2, wherein

the detection device includes an imaging unit that records the operation of the robot device, and
the intermediate progress data includes video data of the operation of the robot device recorded by the imaging unit.

6. The cell culture experiment system according to claim 1, further comprising:

a detection device configured to detect information on the operation of the robot device, wherein
the detection device includes a force sensor that detects a magnitude of a force accompanying the operation of the robot device, and
the intermediate progress data includes data representing the magnitude of the force obtained by the force sensor.

7. The cell culture experiment system according to claim 6, wherein the force sensor detects a pressure when an instrument handled by the robot device comes into contact with an object.

8. The cell culture experiment system according to claim 2, wherein

at least the control device, the robot device, and the detection device among the plurality of devices constituting the cell culture experiment system are synchronized based on a common clock signal.

9. The cell culture experiment system according to claim 8, wherein
a time error representing accuracy of the clock signal is 10 microseconds or less.

10. The cell culture experiment system according to claim 1, wherein
the recipe determination device divides the plurality of samples into a verification group and an efficiency improvement group, and resets, based on the posterior probability of each of the samples, configurations of the samples in the verification group and the efficiency improvement group such that a group of the samples set with a combination of a type of the environmental parameter considered to be highly likely to contribute to successful cell culture and a value of the parameter has a higher culture success rate, and a group of the samples set with a combination of a type of the environmental parameter considered to be highly likely to contribute to failed cell culture and a value of the parameter has a lower culture success rate.

11. A cell culture experiment method comprising:

setting, by a recipe determination device, a recipe to be applied to each of a plurality of samples of a cell to be cultured;
controlling, by a control device, an operation of a robot device such that the robot device performs a culture experiment on each of the plurality of samples according to the set recipe; and
storing, by a database device, intermediate progress data for each of the samples in a time series manner in a progress of one culture experiment in which a culture result of each of the plurality of samples is determined, wherein
the recipe determination device

analyzes a tendency in culture for each sample based on the intermediate progress data for each sample stored in the database device,
calculates, for each sample, a posterior probability representing a degree of possibility that a value of each of a plurality of environmental parameters that constitute the set recipe causes future successful culture of the sample, based on an analysis result of the tendency in the culture of the sample and a difference in the time-series intermediate progress data of the sample,
determines one or more of the environmental parameters having a relatively high calculated posterior probability as a verification parameter, and
corrects the recipe for each sample by changing a parameter value of the determined verification parameter, and

the control device controls the operation of the robot device such that the robot device continues to perform the culture experiment on each of the plurality of samples according to the corrected recipe.

12. A program causing one or more computers or processors to function as:

a recipe determination device configured to set a recipe to be applied to each of a plurality of samples of a cell to be cultured;
a control device configured to control an operation of a robot device such that the robot device performs a culture experiment on each of the plurality of samples according to the set recipe; and
a database device configured to store intermediate progress data for each of the samples in a time series manner in a progress of one culture experiment in which a culture result of each of the plurality of samples is determined, wherein
the recipe determination device

analyzes a tendency in culture for each sample based on the intermediate progress data for each sample stored in the database device,
calculates, for each sample, a posterior probability representing a degree of possibility that a value of each of a plurality of environmental parameters that constitute the set recipe causes future successful culture of the sample, based on an analysis result of the tendency in the culture of the sample and a difference in the time-series intermediate progress data of the sample,
determines one or more of the environmental parameters having a relatively high calculated posterior probability as a verification parameter, and
corrects the recipe for each sample by changing a parameter value of the determined verification parameter, and

the control device controls the operation of the robot device such that the robot device continues to perform the culture experiment on each of the plurality of samples according to the corrected recipe.

[FIG. 1]

LOCAL BUS LB          CLOUD NETWORK BUS CB

STERILE OPERATION AREA SC1

DETECTION DEVICE — 400

IMAGING UNIT — 401

SENSOR UNIT — 402

⇕

ROBOT DEVICE — 300

⇕

600

CELL CULTURE APPARATUS

601          602          603

CULTURE UNIT | MEASUREMENT UNIT | CHEMICAL SOLUTION INSTALLATION UNIT

GENERAL MANAGEMENT AREA SC3    200

TERMINAL DEVICE

100: CONTROL DEVICE    102

DETECTION CONTROL UNIT

OPERATION CONTROL UNIT

201          101

TERMINAL DEVICE

STERILE OPERATION AREA SC2

ANOTHER AREA SC4

550

RECIPE DETERMINATION DEVICE

DATABASE DEVICE — 500

EP 4 779 010 A1

10

[FIG. 2]

[FIG. 3]

[FIG. 4]

[FIG. 5]

START

INITIAL SETTING OF
DETERMINATION RECIPE — S101

D ← → B

CALCULATION OF LIKELIHOOD — S102

CALCULATION OF POSTERIOR PROBABILITY — S103

CORRECTION OF DETERMINATION
RECIPE → n-TH DETERMINATION RECIPE — S104

THERE IS CHANGE IN STATE OF
SAMPLES OF VERIFICATION GROUP? — S105
No →

Yes

S106 — RESET CONFIGURATIONS
OF VERIFICATION GROUP
AND EFFICIENCY
IMPROVEMENT GROUP

MAINTAIN CONFIGURATIONS
OF VERIFICATION GROUP
AND EFFICIENCY
IMPROVEMENT GROUP — S107

START EXPERIMENT — S108

FINAL RESULT OF CULTURE
EXPERIMENT OBTAINED? — S109
Yes → A

No

C

[FIG. 6]

S118

EXAMINE CAUSAL RELATIONSHIP
BETWEEN CAUSE AND
INTERMEDIATE PROGRESS DATA
FOR TARGET SAMPLE, ADD
EXAMINATION RESULT TO RESULT
INDEX, AND STORE RESULT INDEX

STORE RESULT INDEX
FOR TARGET SAMPLE

S119

No
B ← END EXPERIMENT?

S116

Yes

END

S114

A → CHECK CULTURE RESULTS
OF VERIFICATION
GROUP AND EFFICIENCY
IMPROVEMENT GROUP

S115

$m >$ SUCCESS INDEX $> n$?

Yes

No S117

RESEARCHER
CHECKS CAUSE

[FIG. 7]

(D)

| STORE INTERMEDIATE PROGRESS DATA FOR EACH SAMPLE | ⟵ S113 |

(C)

S110(-1)

S110(-k)

| ACQUIRE VIDEO DATA IN PREDETERMINED OPERATION PROCESS PERIOD | · · · | ACQUIRE ENVIRONMENTAL DATA IN PREDETERMINED OPERATION PROCESS PERIOD |

No — THERE IS CHARACTERISTIC IN LIGHT OF REFERENCE?

No · · · — THERE IS CHARACTERISTIC IN LIGHT OF REFERENCE?

Yes

Yes

S111(-1)

S111(-k)

| ADD MARKING INDICATING CHARACTERISTIC TO VIDEO DATA | · · · | ADD MARKING INDICATING CHARACTERISTIC TO ENVIRONMENTAL DATA |

S112(-1)

S112(-k)

[FIG. 8]

[FIG. 9]

CELL

ENZYMATIC TREATMENT

DAMAGED CELL

SCRAPING

SCAFFOLDING AGENT

[FIG. 10]

CLOCK DEVICE
ACCURACY ≤ 10 μS

103

100

300

400

200,201

CONTROL DEVICE

ROBOT DEVICE

DETECTION DEVICE

TERMINAL DEVICE

[FIG. 11]

H11

EXAMPLE OF PART OF INTERMEDIATE PROGRESS DATA OF SAMPLE No. 1

| INTERMEDIATE PROGRESS DATA No. | START TIME | END TIME | VIDEO NUMBER |
|---|---|---|---|
| H111 | 23'35"500 | 23'35"501 | VD164 |
| H112 | 25'23"420 | 25'23"423 | VD178 |
| H113 | 30'08"004 | 30'08"006 | VD186 |
| H114 | 42'12"268 | 42'12"269 | VD1126 |
| ⋮ | ⋮ | ⋮ | ⋮ |

[FIG. 12]

300a

PA1

402a

402b

PA2

[FIG. 13]

F13a

F13b

F13c

661    663

666    PA4

310    311
668

PA5    PA5

PA3

664

662

665

667

[FIG. 14]

H14

EXAMPLE OF PART OF INTERMEDIATE PROGRESS DATA OF SAMPLE No. 1

| INTERMEDIATE PROGRESS DATA No. | DETECTED PRESSURE | | | CASE (PROCESS) |
|---|---|---|---|---|
| | x AXIS | y AXIS | z AXIS | |
| H141 | 12.4 | 2.3 | 25.7 | PEEL ADHERENT CELL |
| H142 | 4.4 | 4.4 | 0 | TAKE OUT SPECIMEN |
| H143 | 15.1 | 15.1 | 7.2 | OPEN TEST TUBE LID |
| H144 | 15.1 | 15.1 | 7.2 | CLOSE TEST TUBE LID |
| ⋮ | ⋮ | ⋮ | ⋮ | ⋮ |

[FIG. 15]

H15

EXAMPLE OF PART OF INTERMEDIATE PROGRESS DATA OF SAMPLE No. 1

| INTERMEDIATE PROGRESS DATA No. | CORRECTION TIMING | CULTURE RECIPE (PARAMETER SET) | LIKELIHOOD | POSTERIOR PROBABILITY |
|---|---|---|---|---|
| H151 | T11 | RD11 | L11 | PP11 |
| H152 | T12 | RD12 | L12 | PP12 |
| H153 | T13 | RD13 | L13 | PP13 |
| H154 | T14 | RD14 | L14 | PP14 |
| H155 | T15 | RD15 | L15 | PP15 |
| ⋮ | ⋮ | ⋮ | ⋮ | ⋮ |

[FIG. 16]

DISTRIBUTION OF LIKELIHOOD FUNCTION

[FIG. 17]

LIKELIHOOD FUNCTION REPRESENTING RELATIONSHIP BETWEEN PRIOR PROBABILITY DISTRIBUTION AND LIKELIHOOD

| p | 0.1 | 0.2 | 0.3 | 0.4 | 0.5 | 0.6 | 0.7 | 0.8 | 0.9 | SUM |
|---|-----|-----|-----|-----|-----|-----|-----|-----|-----|-----|
| PRIOR PROBABILITY | 0.11 | 0.11 | 0.11 | 0.11 | 0.12 | 0.11 | 0.11 | 0.11 | 0.11 | 1 |
| LIKELIHOOD | 0.090 | 0.22 | 0.13 | 0.035 | 0.0046 | 0.00027 | 0 | 0 | 0 | 0.48 |

[FIG. 18]

DISTRIBUTION OF POSTERIOR PROBABILITY

## INTERNATIONAL SEARCH REPORT

| International application No. |
| --- |
| **PCT/JP2024/024976** |

**A.      CLASSIFICATION OF SUBJECT MATTER**

*C12M 1/00*(2006.01)i; *C12M 3/00*(2006.01)i; *C12N 1/00*(2006.01)i; *C12N 5/07*(2010.01)i
FI:    C12M1/00 A; C12M3/00 Z; C12M3/00 A; C12N1/00 C; C12N5/07

According to International Patent Classification (IPC) or to both national classification and IPC

**B.      FIELDS SEARCHED**

Minimum documentation searched (classification system followed by classification symbols)

C12M1/00; C12M3/00; C12N1/00; C12N5/07

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.      DOCUMENTS CONSIDERED TO BE RELEVANT**

| Category* | Citation of document, with indication, where appropriate, of the relevant passages | Relevant to claim No. |
| --- | --- | --- |
| A | CN 112662551 A (SHANGHAI YAOMING BIO-MEDICINE CO., LTD.) 16 April 2021 (2021-04-16) | 1-12 |
| A | JP 2021-153533 A (YASKAWA ELECTRIC CORP.) 07 October 2021 (2021-10-07) | 1-12 |
| A | US 2023/0168662 A1 (THE AUTOMATION PARTNERSHIP (CAMBRIDGE) LIMITED) 01 June 2023 (2023-06-01) | 1-12 |
| A | JP 2023-035238 A (EPISTRA INC.) 13 March 2023 (2023-03-13) | 1-12 |

☐ Further documents are listed in the continuation of Box C.          ☑ See patent family annex.

| | |
| --- | --- |
| *        Special categories of cited documents: | "T"    later document published after the international filing date or priority date and not in conflict with the application but cited to understand the principle or theory underlying the invention |
| "A"    document defining the general state of the art which is not considered to be of particular relevance | |
| "D"    document cited by the applicant in the international application | "X"    document of particular relevance; the claimed invention cannot be considered novel or cannot be considered to involve an inventive step when the document is taken alone |
| "E"    earlier application or patent but published on or after the international filing date | |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | "&"    document member of the same patent family |
| "P"    document published prior to the international filing date but later than the priority date claimed | |

| Date of the actual completion of the international search | Date of mailing of the international search report |
| --- | --- |
| **12 September 2024** | **24 September 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
**Information on patent family members**

International application No.

**PCT/JP2024/024976**

| Patent document cited in search report | | | Publication date (day/month/year) | Patent family member(s) | | | Publication date (day/month/year) |
|---|---|---|---|---|---|---|---|
| CN | 112662551 | A | 16 April 2021 | (Family: none) | | | |
| JP | 2021-153533 | A | 07 October 2021 | US | 2021/0301239 | A1 | |
| | | | | EP | 3888857 | A1 | |
| | | | | CN | 113462562 | A | |
| US | 2023/0168662 | A1 | 01 June 2023 | WO | 2021/214091 | A1 | |
| | | | | EP | 3901710 | A1 | |
| | | | | CN | 115335781 | A | |
| | | | | KR | 10-2022-0166813 | A | |
| JP | 2023-035238 | A | 13 March 2023 | WO | 2023/033005 | A1 | |
| | | | | CN | 117858953 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023035238 A